# EUROPEAN PATENT APPLICATION

(11) **EP 1 772 104 A2**
(43) Date of publication of application: **11.04.2007**
(21) Application number: 06021068.9
(22) Date of filing: 06.10.2006
(51) Int. Cl.: A61B 10/02, A61B 17/34

(54) **Contamination protection device and method for use**

(30) Priority: 07.10.2005 US 596626 P
(71) Applicant: Tsion Israel Medical Systems Ltd., 95464 Jerusalem (IL)
(72) Inventor: Rahamimov, Nimrod Tsvi, Dr., Ganei Tikva 55900 (IL)
(74) Representative: Schorr, Frank Jürgen

(57) **Abstract**

A multi-purpose contamination protection device includes a first generally tubular member defining a lumen therethrough having a distal opening, and a second generally tubular member capable of selectively assuming a first disposition in generally overlaying and proximal relationship with respect to at least a part of said first tubular member and a second disposition in which the second tubular member is inverted inside out with respect to the first disposition and in a generally distal relationship with respect to said part of said first tubular member. Methods of use are also disclosed.

A multi-purpose contamination protection device includes a first generally tubular member defining a lumen therethrough having a distal opening, and a second generally tubular member capable of selectively assuming a first disposition in generally overlaying and proximal relationship with respect to at least a part of said first tubular member and a second disposition in which the second tubular member is inverted inside out with respect to the first disposition and in a generally distal relationship with respect to said part of said first tubular member. Methods of use are also disclosed.

## Description

### FIELD OF THE INVENTION

This invention relates to methods and devices used for protection from contamination, especially with probe-like elongate instruments, in particular for performing biopsies and the like.

### BACKGROUND OF THE INVENTION

Biopsy refers to the removal of a tissue or cell sample, from humans or animals, typically for the purpose of examination and/or culture, which may assist in the medical diagnosis of the tissue or sample, and/or in formulating a treatment. Some types of biopsies are performed for the purpose of obtaining a healthy sample for transplant tissue-type matching evaluation. In some forms of biopsy, a biopsy device, such as a biopsy needle, for example, is inserted into the suspected site in the body, and the cells/tissue sample is extracted via the needle using a syringe. Where the site is located in the body such that the needle has to traverse other tissue layers, retraction of the needle may contaminate these other tissue layers with cells or other biological material from the site. This may be of particular concern with cancer patients, where such contamination may cause seeding of cancer cells elsewhere in the body, which may worsen a patient's prognosis.

In oncology surgery, special attention is given to the biopsy tract, through which the biopsy was obtained, and the tract is normally excised along with the tumor to prevent diseased cells remaining in the body. For this purpose, the biopsy to be performed along special trajectories so that total excision of the tract is possible.

Examples of special attention to biopsy tract planning and excision can be found in the field of limb-salvage surgery, where only specially trained surgeons are encouraged to perform the biopsy. These surgeons will insert the biopsy device through muscular compartments that are expendable, and can be sacrificed during the definitive surgical procedure that will follow. In other areas of the body such as the kidneys, where it is not possible to plan a safe trajectory, total amputation of the organ is preferred to biopsy, since a clean tract can not be guaranteed.

The following publications are presented by way of general background interest.

US 6,582,426 discloses a modular biopsy, ablation and track coagulation needle apparatus that is intended to allow the biopsy needle to be inserted into the delivery needle and removed when not needed, and that is intended to allow an inner ablation needle to be introduced and coaxially engaged with the delivery needle to biopsy a tumor, ablate it and coagulate the track through ablation.

US 2005/203439 discloses a biopsy device for taking tissue samples, includes a housing, a removable element and a control panel. The housing contains an electric power source and a tension slide connected to the power source. The removable element is configured for insertion into the housing and includes a biopsy needle unit, a vacuum pressure-generating device and a control panel. The biopsy needle unit can be arranged on the tension slide and includes a hollow biopsy needle with a sample removal chamber and a cutting sheath.

US 2005/165328 discloses a biopsy device for tissue collection having a housing and a removable element. A power source is contained within the housing and the removable unit includes a biopsy needle module and a pressure source. The biopsy needle module includes a biopsy needle and a cutting sleeve, the biopsy needle having a sharpened distal end and a distal opening for collection of tissue, the cutting sleeve having a cutting blade on its distal end.

US 2006/116603 discloses a biopsy device including a catching member for catching and releasing a living sample, a sheath through which the catching member is able to be inserted, and a container that stores the living sample.

US 2005/101879 discloses a needle aspiration biopsy device including a syringe, a valve coupler for controlling a vacuum in the syringe barrel, and an anti-reflux head.

US 2005/090762 discloses an electrosurgical biopsy device including a stylet and a cannula movably mounted on a base. The stylet has a shaft with a head at its distal end and a stylet ablation element extending distally from the head, and the stylet shaft is disposed through the cannula for axial translation therein between withdrawn and extended positions.

US 2004/097829 discloses a tissue biopsy and processing device including a biopsy member adapted to remove a tissue sample from a patient's body, and a processing apparatus adapted to receive the tissue sample from the biopsy device, and to dissociate and mince the tissue sample in preparation for further use.

US 2004/059253 discloses an apparatus and methods for obtaining biopsy samples using an endoscope assembly. In one embodiment, an assembly adapted for use with an endoscopic insertion tube includes a sheath having a body portion adapted to at least partially encapsulate a distal portion of the insertion tube, and a biopsy sampling device attached to the sheath and including a collection member proximate an end of the body portion.

US 2003/097079 discloses a sheath for a biopsy needle for removing a sample from a living body, the sheath comprising a substantially tubular structure configured to accommodate at least a portion of a biopsy needle. The tubular structure has a side wall, a first end opening which in use remains outside of the body, and a second end opening which in use is inserted into the body. The tubular portion has a length between the first and second openings which is greater than the distance between sample to be removed and the outside of the body.

US 2003/040681 discloses a biopsy apparatus including: a body section having an opening at a distal end thereof; a sample collection means for obtaining a biopsy sample; and a first actuator to provide relative movement between the sample collection means and the body section wherein the sample collection means is operable between a first, retracted position and a second, extended position.

### SUMMARY OF THE INVENTION

Herein the term "proximal" refers to directions towards the user of the device and away from the site with respect to which the device is being used to perform a biopsy, for example. Conversely, the term "distal" refers to directions away from the user of the device and towards the site with respect to which the device is being used to perform a biopsy, for example.

The present invention relates to a multi-purpose contamination protection device comprising a first elongate member defining a lumen therethrough having a distal opening, and further comprising a second member capable of selectively assuming a first disposition in generally enveloping and proximal relationship with respect to at least a part of said first member and a second disposition in which the second member is inverted inside out with respect to the first disposition and in a generally distal relationship with respect to said part of said first tubular member. At least a part of an outward-facing surface of the second member, in the first disposition, is inverted in the second disposition to become inward-looking with respect to at least a part of the first member.

According to some embodiments of the invention, the multi-purpose contamination protection device comprising a first generally tubular member defining a lumen therethrough having a distal opening, and further comprising a second generally tubular member capable of selectively assuming a first disposition in generally overlaying and proximal relationship with respect to at least a part of said first tubular member and a second disposition in which the second tubular member is inverted inside out with respect to the first disposition and in a generally distal relationship with respect to said part of said first tubular member.

According to some embodiments of the invention, the multi-purpose contamination protection device may comprise:
a tube member comprising a tube outer surface and an inner lumen providing open communication between a distal opening and a proximal opening thereof, the lumen adapted for enabling a suitable elongate instrument to be inserted therethrough to perform a function at or beyond said distal opening;
a sleeve member comprising a first end sealingly connected to said tube outer surface at a connection station and further comprising a second open end,
wherein said sleeve is configured for being inverted inside out such as to enable the device to be operated between an inserting configuration and a retracting configuration, wherein:
in the inserting configuration at least a portion of the sleeve extends in a generally overlaying relationship with respect to a portion of the said tube outer surface proximal to said connection station, such that a first surface of said at least one portion of said sleeve is outwardly facing with respect to said tube member, and wherein
in the retracting configuration, said at least one portion of the sleeve is inverted to assume a position distal of said connection station, wherein said first surface is inwardly facing with respect to said tube member.

The present invention relates to a multi-purpose contamination protection device comprising a first, typically elongate, member defining a lumen therethrough having a distal opening, and further comprising a second member capable of selectively assuming a first disposition in generally enveloping and proximal relationship with respect to at least a part of said first member and a second disposition in which the second member is inverted inside out with respect to the first disposition and in a generally distal relationship with respect to said part of said first member. In said first disposition, there is open communication between an outside of the device and the lumen via said distal opening. In some embodiments:
said first member comprises a tube member comprising a tube outer surface and said lumen providing open communication between said distal opening and a proximal opening thereof, said lumen adapted for enabling a suitable elongate instrument to be inserted therethrough to perform a function at or beyond said distal opening;
said second member comprising a sleeve member having a first end connected to said tube outer surface at a connection station and further comprising a second open end,
   wherein said sleeve member is configured for being inverted inside out such as to enable the device to be operated between an inserting configuration and a retracting configuration, wherein:
   in the inserting configuration said sleeve is in said first disposition and at least a portion of the sleeve extends in a generally overlaying relationship with respect to a portion of the said tube outer surface proximal to said connection station, such that a first surface of said at least one portion of said sleeve is outwardly facing with respect to said tube member, and wherein
   in the retracting configuration, said sleeve is in said second disposition and said at least one portion of the sleeve is inverted to assume a position distal of said connection station, wherein said first surface is inwardly facing with respect to said tube member.

The sleeve may be configured for being inverted inside out responsive to the device being operated from an inserting configuration to a retracting configuration.

The sleeve may be generally coaxial with said tube member. The tube member may be substantially rigid in some embodiments, and in other embodiments may be semi-rigid or non-rigid, while the sleeve member is substantially flexible and may be elastically deformable.

Optionally, the distal opening may be adapted for creating a passageway in a material, for example a tissue, by means of rotating and/or pushing the tube distally into the material or tissue. Optionally, the distal opening comprises any one of a sharp annular edge or a serrated annular edge.

Optionally, the sleeve member may be sealingly connected to said tube outer surface via a suitable ring. Optionally, the sleeve member may be non-sealingly connected to said tube outer surface via a suitable ring.

Optionally, and in some embodiments, in the inserting configuration the full axial length of the sleeve extends in a generally overlaying relationship with respect to a portion of the said tube outer surface proximal to said connection station.

Optionally, and in some embodiments, said device comprises a lining housing concentrically mounted on said tube outer surface, and said sleeve is in the form of a deployable lining anchored at said housing. The lining may comprise an anchoring flange adapted for anchoring the lining at a proximal position as the device is inserted in a distal direction. The lining may be adapted for being progressively deployed as the device is inserted in a distal direction. In the inserting configuration, at least a portion of said lining may be accommodated in said lining housing in a folded configuration.

The device further comprises an arrangement for preventing proximal sliding of the sleeve with respect to a passageway into which the device is inserted. Optionally, the arrangement comprises suitable projections projecting in a generally proximal direction and adapted to anchor into said passageway. Alternatively or additionally, the arrangement comprises any one of fiber or adhesives.

By way of non-limiting example, the tube member may be made from any one of: stainless steel, Titanium, plastics, carbon fiber, composite materials and synthetic materials. By way of non-limiting example, the sleeve may be made from any one of: silicone, Latex, mesh fibers and various synthetic materials.

The device according to the invention may be adapted for use with a biopsy medical instrument, and/or for enabling any one of a syringe needle, a biopsy needle, and the like to be inserted through said lumen.

Optionally, said sleeve comprises a sedative or disinfection material.

According to other aspects of the invention, a method is provided for performing a medical procedure at a target tissue of a body, comprising:
(a) providing a device according to the invention;
(b) inserting said device into a body tissue such that said distal opening of said tube member is proximal to or embedded in said target tissue, and said sleeve is radially pressed against tissue walls at least in an intervening tissue layer between the target tissue and the entry point of said device with respect to said body;
(c) performing said medical procedure via said lumen; and
(d) pulling said tube member in a proximal direction and allowing the tube member to invert the said sleeve until the device is fully removed from the body.

Optionally, said distal opening of the device comprises a sharp or serrated annular edge, and in step (b) the device is manipulated so as to form a passageway into said tissue layer and said target tissue as said device is inserted thereinto.

Optionally, the said procedure is a biopsy of said target tissue, and step (c) comprises inserting a suitable biopsy member through said lumen so that a tissue capturing portion of the biopsy member is in contact with said target tissue, procuring a tissue sample, and removing said biopsy member via said lumen.

Optionally, step (c) comprises any suitable biopsy sampling procedure optionally including the following procedures: core, fine-needle, Basket, Punch, Pituitary, Flat blade, Vacuum assisted, Endoscopy, Tru-cut and so on.

Optionally, said procedure is one of a therapeutic or diagnostic procedure with respect to said target tissue, and step (c) comprises inserting therapeutic or diagnostic substances, materials or devices into said target tissue via said lumen. Further optionally, step (c) includes the insertion of brachitherapy needles or rods, or the insertion of a controlled release chemotherapy capsule.

According to other aspects of the invention, a method is provided performing a medical procedure at a target tissue via an intervening tissue layer, comprising:
(i) providing an access channel through said tissue layer to access to said target tissue such that said procedure can be performed, said channel comprising concentric inner and outer walls adapted to prevent contact passage of contaminants therethrough between an inside and an outside of said channel, said inner and outer walls being sealingly joined at a distal station;
(ii) conducting said procedure of said target tissue via said channel; and
(iii) removing said inner wall in a proximal direction while maintaining the outer wall in place, so that the outer wall is progressively turned inside out.

For example, said procedure may include performing a biopsy at a target tissue via an intervening tissue layer, and steps (i) to (iii) comprise:
(i) providing an access channel through said tissue layer to enable a biopsy member to access said target tissue, said channel comprising concentric inner and outer walls adapted to prevent contact passage of contaminants therethrough between an inside and an outside of said channel, said inner and outer walls being sealingly joined at a distal station;
(ii) conducting a biopsy of said target tissue with a suitable biopsy member via said channel and removing said biopsy member; and
(iii) removing said inner wall in a proximal direction while maintaining the outer wall in place, so that the outer wall is progressively turned inside out.

For example, said procedure may include performing a therapeutic or diagnostic procedure at a target tissue via an intervening tissue layer, and steps (i) to (iii) comprise:
(i) providing an access channel through said tissue layer to enable therapeutic or diagnostic substances, materials or devices to be inserted into said target tissue via said lumen;
(ii) inserting therapeutic or diagnostic substances, materials or devices into said target tissue via said lumen; and
(iii) removing said inner wall in a proximal direction while maintaining the outer wall in place, so that the outer wall is progressively turned inside out.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** is a schematic side view of a medical device according to one embodiment of the present invention;
**Fig. 2** is a schematic partial cross sectional view of the device of Fig. 1;
**Figs. 3A** to **3D** are schematic illustrations of the device of Fig. 1 during various stages of its operation;
**Fig. 4** is a schematic side view of a device according to another embodiment of the present invention;
**Fig. 5** is a schematic partial cross section of the device of Fig. 4;
**Figs. 6A** to **6D** are schematic illustrations of the device of Fig. 4 during various stages of its operation; and
**Fig. 7** is a schematic view of an embodiment of a sheath/lining used with the device of Fig. 1 and Fig. 4;
**Fig. 8** is a schematic partial cross sectional view of a variation of the embodiment of Fig. 1.

### DETAILED DESCRIPTION OF EMBODIMENTS

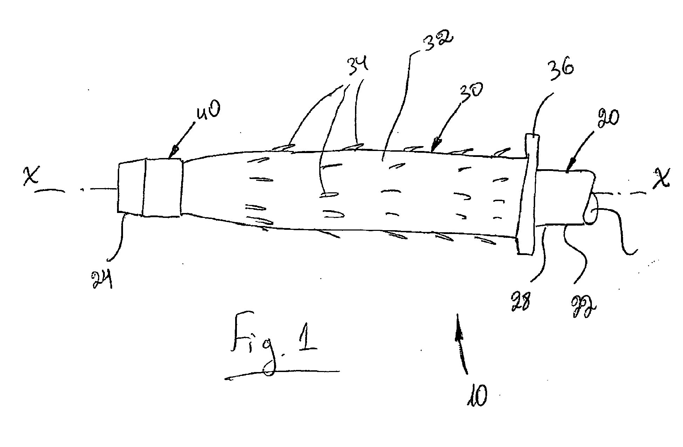

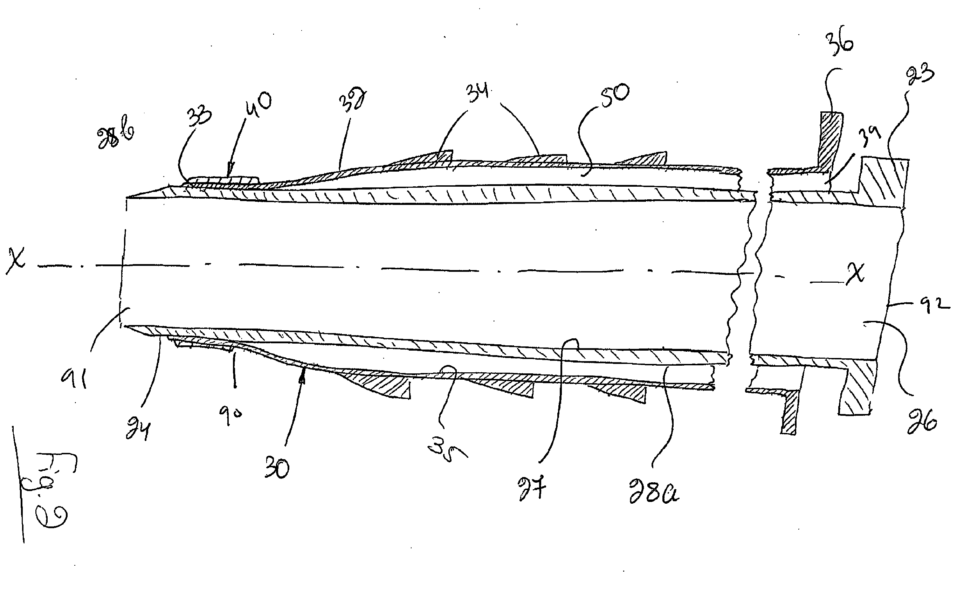

Referring to Fig. **1,** a first embodiment of a medical device, generally designated **10,** adapted for use with a variety of biopsy members, may be in the form of an elongate probe and comprises a tube member **20** and a sleeve in the form of a sheath **30,** which are made from medically compatible materials as will be discussed in greater detail herein. A biopsy member refers to any suitable medical instrument, typically in the form of an elongate probe or the like, that may be used for performing a biopsy at a medical site.

The tube member **20** and a sheath **30** are substantially tubular and generally coaxial with respect to a common axis X. While the cross-sections of the tube member **20** and a sheath **30** are typically circular, they may assume any other suitable shape, for example, oval, elliptical, polygonal etc, as may be medically compatible, and thus the term "tubular" is not restricted to circular cross-sections only. The sheath **30** is sealingly affixed at one end **31** thereof to the tube member **20** at a connecting station **90** by means of a grip ring **40** close to the distal end **22** of the tube member **20,** although a variety of alternative methods may be used for affixing the two components together, as will be described in greater detail herein. In the inserting configuration illustrated in Fig. **1,** the main body of the sheath **30** proximal to the ring **40** is disposed generally concentrically and in overlying relationship with respect to the tube **20,** or at least with respect to a portion of the outer surface **28** extending proximally with respect to connecting station **90.** The main body of the sheath **30** may be freely displaced axially with respect to the outer surface **28** of the tube member **20** and forms an essentially loose sleeve. The sheath **30** comprises a plurality of directional anchoring projections **34** disposed on its outer surface **32,** the function of which will be discussed in detail later.

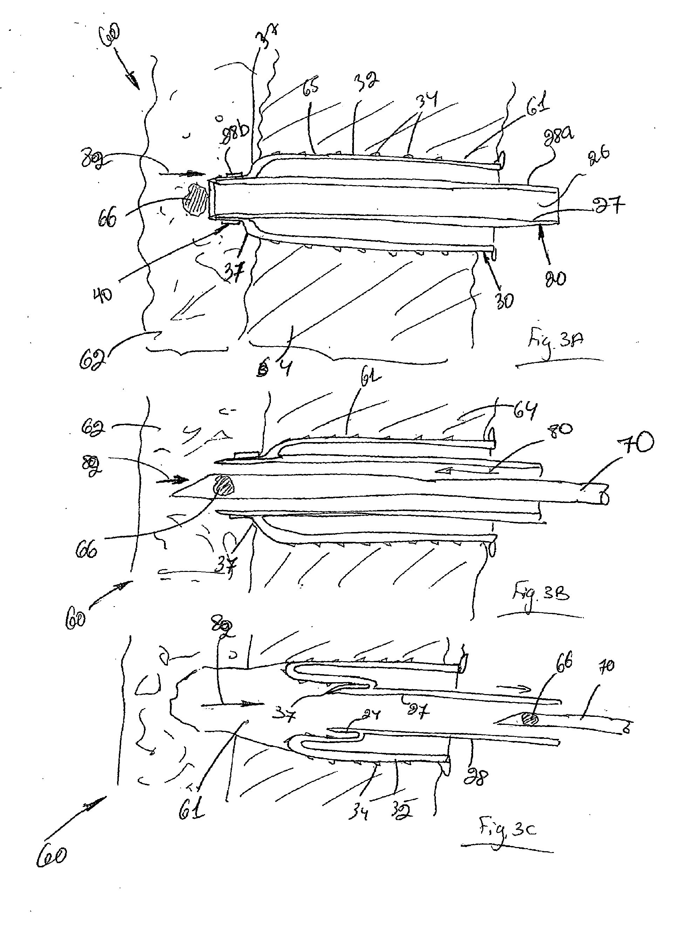

Turning now to Fig. **2,** a cross section of the device **10** is shown. The tube member **20** has a lumen **26,** defined between the distal opening **91** and the proximal opening **92,** adapted for receiving therein a biopsy member, such as for example a syringe needle (not shown in this figure), or indeed any other suitable medical instrument that it may be desired to use with said device **10,** as will be discussed in detail later. The tube member **20** comprises a distal tube flange **23,** and the sheath **30** also comprises a distal sheath flange **36** at the open end **39** of the sheath, positioned adjacent to the flange **23.** Flange **23** may be connectable to a manual grip, or alternatively to a powered drive, to aid in the insertion process, as will be described in more detail herein.

The grip ring **40** grips the distal edge **33** of the sheath **30** (as per the insertion configuration of Fig. **1)** and sealingly presses it against the tube member **20,** thus dividing the outer surface **28** of the tube **28** into a proximal outer surface **28a** extending under the sheath **30** from the connection station **90** proximal to the ring **40** to the flange **23,** and a distal outer surface **28b** extending from the grip ring **40** to the tip **24** of the tube **20.** The inner surface **27** extends throughout the entire length of the lumen **26.**

The connection between the sheath 30 and the tube member **20** may be achieved by various means other than a grip ring **40,** for example via an adhesive, an internal ring, welding, etc. Alternatively, both tube member **20** and sheath **30** may be integrally formed from a suitable material, optionally treated or constructed so that the tube **20** may be substantially rigid whereas the sheath portion **30** may be constructed or treated to be substantially flexible.

Thus the tube **20** may be formed as a relatively rigid member, and may have a rectilinear central axis X. In other embodiments, the tube **20** may be curved longitudinally or otherwise contoured. In yet other embodiments, the tube may be formed as a semi-rigid or non-rigid member, and stiffness therefore while in the insertion configuration may be provided by means of an obturator or the like.

On the other hand, the sheath **30** may be formed as a flexible, elastic and/or stretchable member, in particular such as to be invertible from the concentric configuration shown in Figs **1** and **2** with respect to the inner tube **20,** wherein the sheath **30** at least partially envelopes the inner tube **20,** to an involuted inside-out configuration, wherein the previously concave, inner-facing surface **35** is now exposed and is convex and outward facing with respect to the inner tube **20.**

Optionally, the tube member **20** and sheath **30** are loosely fitted over one another in the insertion configuration illustrated in Figs. **1** and **2,** such as to permit a lubricant **50** to be provide lubrication between the inner surface **35** of the sheath **30** and the outside surface **28** of the tube **20.**

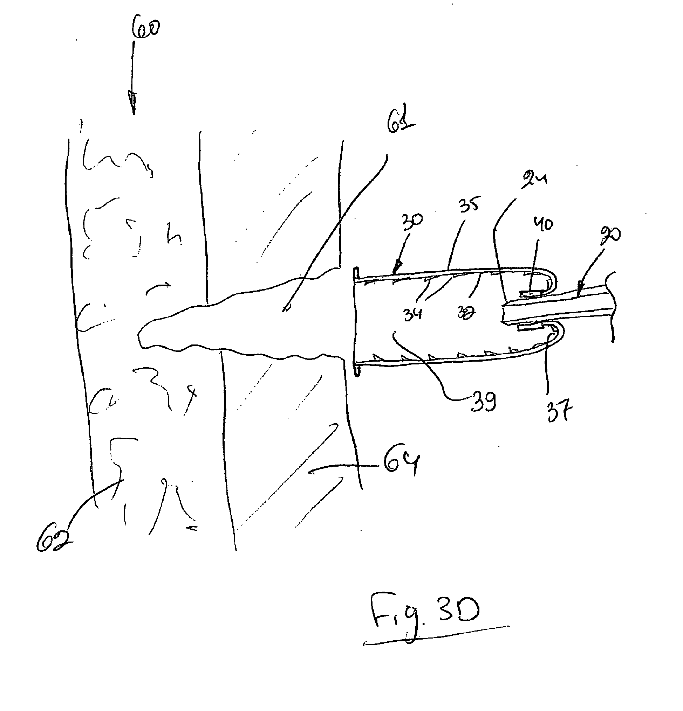

Referring to Figs. **3A** to **3C,** various stages of a biopsy procedure or the like are shown using device **10,** referring to a human or an animal body **60** containing a target tissue **62** separated from the skin or other interface by means of tissue layer **64,** which may comprise any number of different tissues. According to an aspect of the invention, the device **10** may be used in a biopsy procedure, such that a sample **66** of the target tissue **62** may be obtained while minimizing or eliminating the risk of contamination of the clean tissue layer **64** with biological material from the target tissue **62** during the procedure.

At the first stage of the procedure depicted in Fig. **3A,** the device **10** in its insertion configuration is inserted through the clean tissue **64** to reach the target tissue **62,** creating a passage **61** traversing the tissue layer **64.** The tip **24** of the tube **20** may be designed as a sufficiently sharp or serrated edge and of an appropriate shape, for example annular, so as to create the passage **61** by itself, for example by the user pressing and/or turning the tube **20** while inserting the device **10.** For this purpose the flange **23** connected to a suitable handle or powered drive, or alternatively the flange **23** may be formed as a handle or a grip providing easier manipulation of the tube member **20,** and aiding in insertion of the device **10** during the penetration stage of the procedure and pulling back the tube member **20** during the removal stage of the procedure.

Alternatively, a suitable boring instrument may be inserted into the lumen **26** such that a cutting or boring tip extends distally from the distal end **24** of the tube, and this instrument produces the passage. Such an instrument may be of particular use when the tube **20** is formed as a non-rigid member.

Alternatively, the passageway may be formed with a suitable instrument, which is then removed and the device inserted in the formed passageway.

Thus, the outer surface **32** of the sheath **30** is in sliding contact with the passageway in a distal direction during the insertion procedure.

The device **10** is inserted into and penetrates the body **60** until the tip **24** of the tube member **20** reaches the target tissue **62,** and the only contact between the device **10** and the clean tissue layer **64** is solely through the outer surface **32** of the sheath **30.** In this position the projections **34** are pointing in direction of arrow **82,** in a generally proximal direction. Therefore, should the device **10** be pulled back in the proximal direction, the projections **34** act as anchoring members and are adapted to prevent the outer surface **32** of the sheath **30** from sliding against the inner surface **65** of the clean tissue layer **64** in direction of arrow **82,** i.e., proximally. In addition, the tip **24,** grip ring **40** and a distal surface portion **37** of the outer surface **32** of the sheath **30** are in contact with the target tissue **62,** and may be contaminated.

The device **10** may be inserted distally until at least a distal part of the sheath **30** penetrates into the target tissue, which may minimize further the risk of contamination when the device is pulled out. Alternatively, the device **10** may be inserted distally until the distal part of the sheath is just proximal of the target tissue sampled, without contact with it. The latter case may be of particular use when the target tissue is surrounded by the same type of tissue, for example the target tissue being a suspected liver tumor, surrounded by healthy liver tissue.

At the second stage of the procedure depicted in Fig. **3B,** a syringe, sampling probe or other biopsy member **70** is inserted into the lumen **26** of the device **10** in direction of arrow **80,** to reach the target tissue **62** and obtain sample **66** therefrom without coming in contact with the clean tissue layer **64.**

Once the sample **66** has been obtained, the biopsy member **70** is pulled back proximally in direction of arrow **82** and removed from the device **10.** The sample **66** may now be analyzed or dealt with according to the appropriate purpose for which it was obtained. Thus, at the next stage of the procedure depicted in Fig. **3C,** the device **10** is removed such as to reduce or eliminate the risk of contaminating the clean tissue **64** with biological material of the target tissue **62** which may be present on the tip **24,** grip ring **40** and the distal surface portion **37** of the sheath **30.**

As also depicted in Fig. 3C, the tube member **20** is pulled out in a proximal direction, and initially most of the sheath **30** remains attached to the clean tissue **64** by virtue of anchoring action of the projections **34;** the distal surface portion **37** of the sheath **30** that was in the target tissue **63** beings to invert so that it faces inwardly, and the tip **24** and grip ring **40** are prevented from coming in contact with the clean tissue **64.** As the tube member **20** is progressively pulled in the proximal direction, due to the connection of the sheath **30** to the tube member **20** using the grip ring **40,** the sheath **30** is progressively peeled off from the clean tissue **64** and undergoes inversion, such that more and more of the external surface **32** of the sheath **30** finds itself on the inside. During this process, the projections **34** serve to prevent any significant proximal sliding of the sheath **30** with respect to the passage, which could otherwise result in the tip **24** and other distal parts of the device **10** from coming into contact with the clean tissue layer **64.**

At the end of the procedure, depicted by Fig. **3D,** the device **10** is completely removed from the body **60,** the sheath **30** may be completely inverted so that the previously outer facing surface **32** of the insertion configuration is now the inner facing surface of the extracted configuration, and conversely the inner facing surface **35** of the insertion configuration is now the outer surface of the extracted configuration. Distal parts of the device **10** that were potentially contaminated with biological material from the target tissue **62,** e.g. the tip **24** of the tube member **20,** the grip ring **40** and the distal surface portion **37,** are now within the hollow **39** created by the inversion of the sheath **30,** making it, *inter alia,* much safer to handle the device **10** after the end of the procedure.

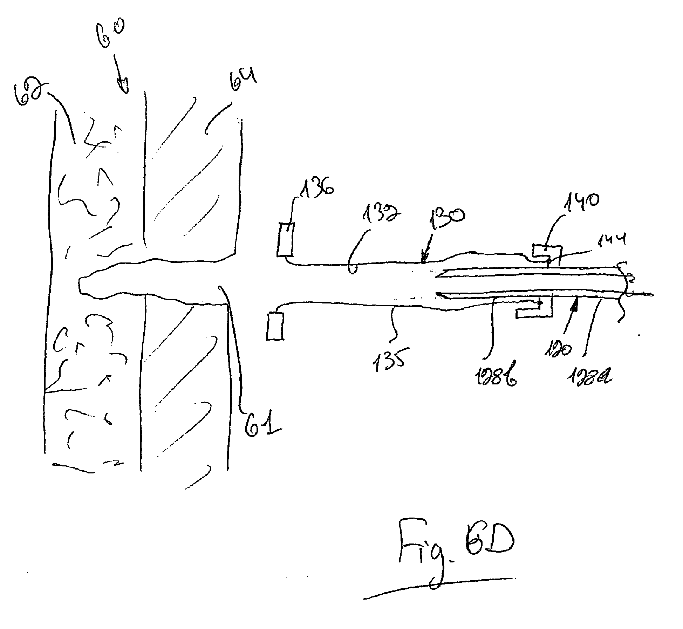

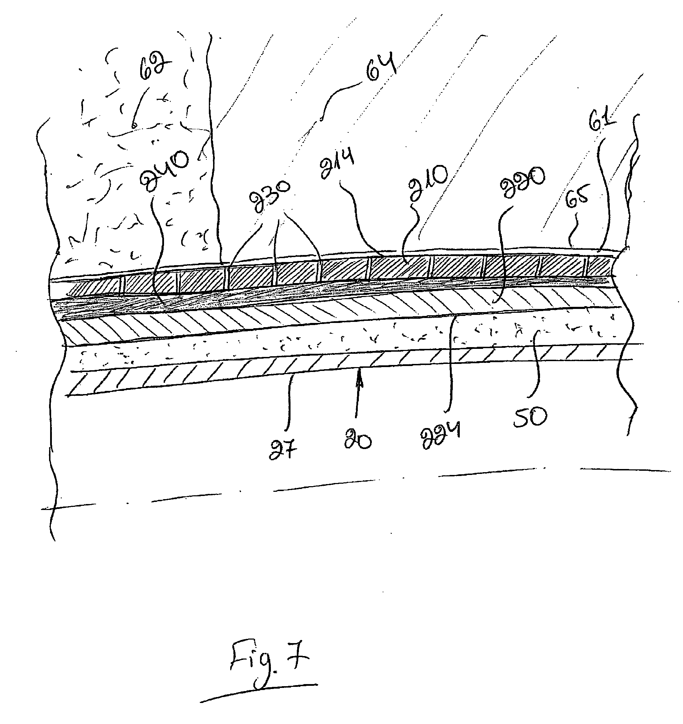

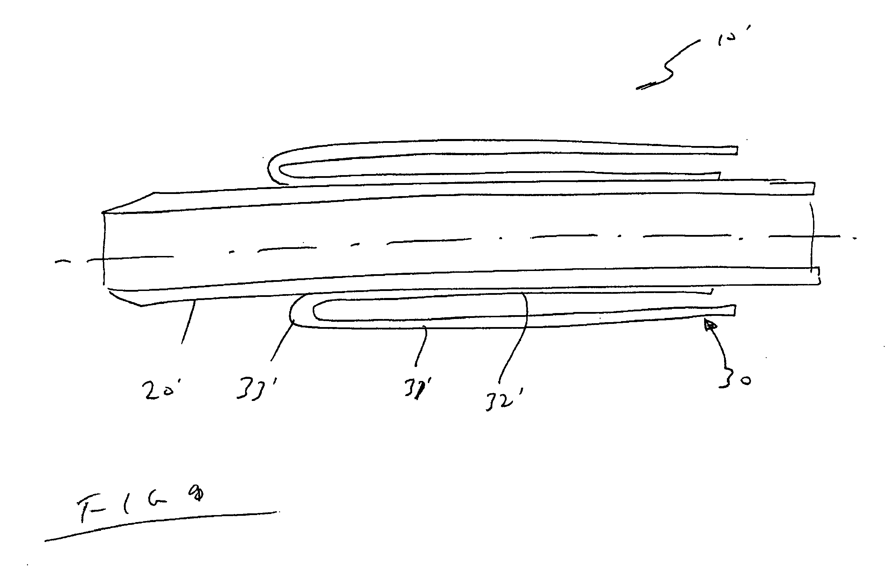

A variation of the first embodiment is illustrated in Fig. **8,** in which the device, designated **10',** comprises an inner tube member **20',** substantially similar to that described for the first embodiment, *mutatis mutandis.* The device **10'** further comprises a sleeve **30'** made from an elastic and flexible material in the shape of a hollow tube comprising a first section **31'** that is folded over the second section **32'** at annular fold **33'.** The second section **32'** may be bonded or otherwise fixed onto the external surface of the tube member **20',** for example by elastically stretching the second section **32'** over the tube member **20'** so that when released the second section **32'** grips the tube member **20'** by mea\ns of the elastic potential energy stored by the second section **32'.** In such a case, the internal diameter of the second section **32',** when unstretched, may be smaller than the external diameter of the tube member **20'.** The device **10'** may be used substantially in the same manner as disclosed for the first embodiment *mutatis mutandis,* and as the device is removed from the target tissue the first section **31'** unfolds such that the annular fold **33'** effectively moves distally with respect to the tube member **20',** and assumes its original tubular form. As with the first embodiment, the outer-facing surface of the second section **32',** when this is folded over the first section **31',** may comprise a suitable anchoring arrangement to prevent or reduce potential proximal slippage between the sheath **30'** and the tissue.

In another aspect of the invention, such a sleeve **30'** rather than be connected to said inner tube, may be mounted directly onto a suitable instrument that is insertable into a body cavity, so as to prevent or minimize contamination of other parts of the body as the instrument is retracted back out of the body.

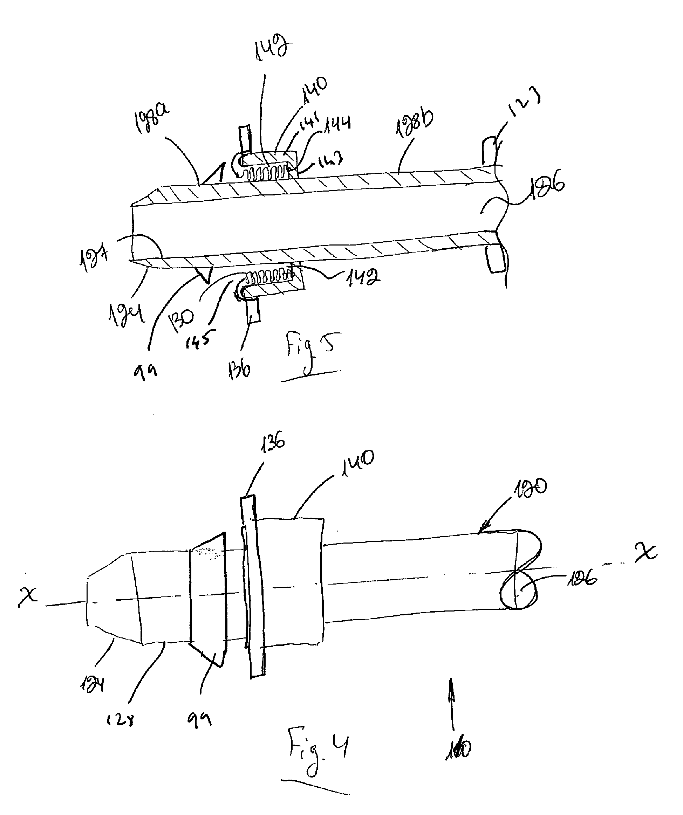

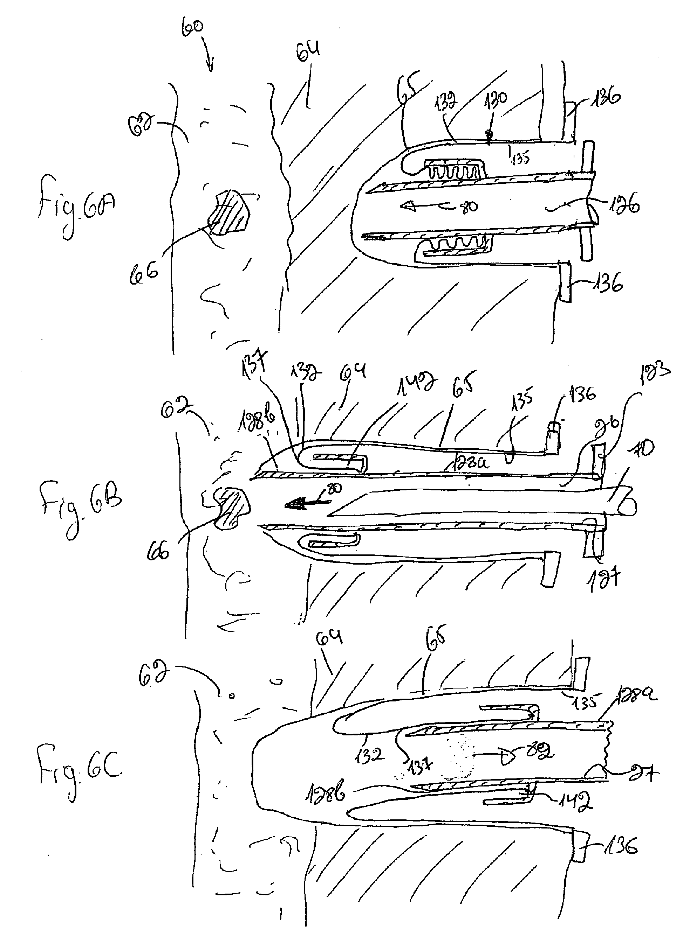

A second embodiment of the device according to the present invention is illustrated in Figs. **4** and **5,** and is generally designated with the numeral **110.** The device **10** comprises a tube member **110,** and a sleeve in the form of a lining **130,** all of which are made of medically compatible materials as for the first embodiment, *mutatis mutandis.* The tube **110** may be similar to that described for the first embodiment, *mutatis mutandis.* On the other hand, the main difference between this embodiment and the first embodiment is that in the first embodiment the sheath is fully deployed proximally when the device is in the insertion configuration, even before being inserted into a body, while in the second embodiment the lining **130** is progressively deployable during insertion.

In the insertion configuration, lining **130** is accommodated in a lining housing **140** in a retracted form, for example comprising accordion-like folds to minimize the axial space taken by the retracted lining. The lining housing **140** may comprise a cylindrical outer wall **141** concentric with and connected to the outer surface **128** of the tube **120** via a housing proximal annular wall **143,** and having an annular opening **145.**

The lining **130** is connected at one end thereof to the inner side of the lining housing **140** at anchor point **142,** which defines a connection station, and at the other end thereof to a flange **136.**

As with the first embodiment, the tube **120** comprises a working channel or lumen **126** adapted to receive therein biopsy member, for example a syringe, or any other suitable medical instrument, and further comprises a flange **123** (seen Fig. **6B)** at the proximal end thereof.

The lining housing **140** essentially divides the outer surface **128** of the tube **120** into a proximal outer surface **128a** extending from the flange **123** to the opening **145** of lining housing **140** or to the anchor point **142,** and a distal outer surface **128b** extending from the lining housing **140** to the tip **124** of the tube **120.**

As with the first embodiments, the lining **130** may be formed as a flexible and stretchable member, in particular such as to be invertible from the concentric configuration shown in Figs. 5 and 6 with respect to the inner tube **120,** to an involuted inside-out configuration, wherein the previously concave, inner-facing surface **135** is now exposed and is convex and outward facing with respect to the inner tube **120.**

Optionally, a deflector **99** may be provided distally of the opening **145** to deflect tissue away from the opening **145** while the device is being inserted into the body. The deflector **99** may comprise an annular member having a wedge-like cross-section, for example.

Referring to Figs. **6A** to **6D,** various stages of a biopsy procedure or the like are shown using device **110,** referring to a human or an animal body **60** containing a target tissue **62** separated from the skin or other interface by means of tissue layer **64,** which may comprise any number of different tissues.

At the first stage of the procedure depicted in Fig. **6A,** the device **110** is inserted through the clean tissue **64** to reach the target tissue **62,** creating a passage **61** within both tissues **62, 64.** The flange **136** is first seated onto the outer surface of tissue **64** at the perimeter of the passage formed therein, and during the penetration of the device **110** into the body **60,** the lining **130** is progressively deployed from the housing **140** as a result of the forward displacement of the tube **120,** since the flange **136** prevents that end of the lining **130** from being dragged into the body and acts as an external anchor. As the lining **130** is deployed, outer surface **132** of the lining **130** progressively covers the inner surface **65** of the passage **61.**

Creation of the passage and penetration of the device **110** into the body may be accomplished in a similar manner to that described for the first embodiment, *mutatis mutandis,* the main difference being that rather than having a sheath sliding distally with respect to the passage formed in the body, the lining is deployed in a generally non-sliding manner with respect to the passage.

The device **110** is inserted into the body **60** until the tip **124** of the lumen **120** reaches the target tissue **62** and the only contact between the device **110** and the clean tissue **64** is via the outer surface **132** of the lining **130.** The tip **124** and especially the outer surface **128b,** and a portion **137** of the outer surface **132** of the lining **130** are in contact with the target tissue **62,** and may be contaminated. The lining **130** may optionally be attached to the clean tissue **64** for example with a suitable temporary adhesive, preventing the outer surface **132** of the lining **130** from sliding against the inner surface **65** of the clean tissue **64** in direction of arrow **82,** and thus inadvertently carrying biological material from the target tissue **62** to the clean tissue **64.**

At the second stage of the procedure depicted in Fig. **6B,** a syringe, sampling probe or other biopsy member **70** is inserted into the lumen **126** in a similar manner to that's described for the first embodiment, *mutatis mutandis,* and once the sample **66** has been obtained, the biopsy member **70** is pulled back in direction of arrow **82** and removed from the device **110,** for further processing.

At the next stage of the procedure depicted in Fig. **6C,** the device **110** is removed from the body in a manner that minimizes or eliminates the risk of potentially contaminating the clean tissue **64** with biological material of the target tissue **62** which may be present on the tip **124,** and on the distal surface portion **137** of the lining **130.** As with the first embodiment, the tube member **120** is pulled out proximally, inverting the lining **130** progressively without inducing any significant sliding between the lining **130** and the passage, thereby containing the possibly contaminated distal parts of the device within the now-inverted and inward facing previously outer facing surface **132** of the lining **130.**

At the end of the procedure depicted by Fig. **6D,** the device **110** is completely removed from the body **60,** the sheath **130** is completely inverted so that the previously outer surface **132** of the insertion configuration is now the inner surface of the extracted configuration, and conversely the inner surface **135** of the insertion configuration is now the outer surface of the extracted configuration. Distal parts of the device **110** that were potentially contaminated with biological material from the target tissue **62,** e.g. the tip **124** of the tube member **20,** and the distal surface portion **137,** are now within the hollow **139** created by the inversion of the lining **130,** making it, *inter alia,* much safer to handle the device **10** after the end of the procedure.

Optionally, with respect to both of the above embodiments, the sheath **30** or lining **130** may be adapted to be covered with a sedative or disinfection material which may eliminate the need for sedation prior to the procedure and/or prevent infections within the passage **61.** Additionally or alternatively, the sheath **30** or lining **130** may be adapted to be covered with an adhesive, which can serve as a cohesive material between the sheath **30** or lining **130** and the surrounding tissue **64.** Thus, an embodiment of such a sheath/lining adapted for use in conjunction with either of the first or second embodiments, is depicted in Fig. **7,** in which the sheath **30** or lining **130** may consist of a first porous layer **210** and a second impermeable layer **220** having a medical substance **240** therebetween. The substance **240** may be an adhesive, sedative, a disinfection material etc. The porous layer **210** has pours **230** disposed along its outer surface **214** through which the substance **240** may be administered to the surrounding tissue **62, 64.**

The insertion and extraction procedures using this variation for the sheath or lining are much like the ones described in the two aforementioned embodiments, *mutatis mutandis.* The impermeable layer **220** still prevents any carrying of biological material from the target tissue **62** to the clean tissue layer **64,** with the exception that the outer surface **214** of the outer layer **210,** is now porous (as opposed to outer surfaces **32, 132** of the previous embodiments) and may allow unwanted biological material to penetrate between the layers. However, this does not affect the procedure since the outer layer **214** will become inverted during the process, much like surface portion **37, 137** of the previous embodiments.

It should be noted here that the tube members **20, 120** may be made from a wide range of materials, for example stainless steel, Titanium, plastics, carbon fiber, composite materials and various synthetic materials. Similarly, the sheath 30 and lining **130** may be made from a wide range of materials including silicone, Latex, mesh fibers and various synthetic materials.

In some variations of the above embodiments, the whole device may be made as a single unitary article, for example from a suitable polymer, part of the device such as the inner tube member being suitably treated to form a rigid component, while another part of the device, corresponding to the sheath or lining may be suitably treated to provide the required flexibility, for example using suitable cross-linking techniques. Alternatively, the difference in rigidity and flexibility between the inner tube and the outer sleeve (sheath or lining) may be provided by suitably designing the device to provide these mechanical properties. For example, the inner tube member may be made with a relatively thick thickness and optionally comprising longitudinal stiffening ribs of the same material (or having hollow ribs into which may be inserted stiffening members, such as for example steel rods or the like), while the outer sleeve portion may be made from substantially thinner thickness of the same material.

The temporary anchoring and non-slip effect provided between the sheath **30** or lining **130** and the passage in the clean tissue layer **64** may be achieved by various methods and means, other than the ones described above. For example, metallic spines, projections or digitations may be provided, made either from the same material as the sheath **30** or lining **130,** or from a different material; fiber strands may be provided; adhesives for example in the form of special coatings such as Polyglactin, Cyanoacrylate, Gelatine etc. may be provided.

The device according to the invention provides for a wide variety of biopsy sampling procedures including: core, fine-needle, Basket, Punch, Pituitary, Flat blade, Vacuum assisted, Endoscopy, Tru-cut etc. Similarly, the device may also be used for many other medical procedures or other non-medical procedures in which it may be desired to obtain access to a particular site in the body or other entity for a particular instrument, for example a medical instrument, and then remove the instrument without possibly contaminating the passageway in the body or entity through which the instrument was passed (via the device of the invention). For example, such an instrument may be a syringe for injecting a medical or other material to a site, or an endoscope for viewing a site, or a surgical instrument (optionally remotely operable) for operating at a site.

The device according to the invention may also be applied to other uses, for example for the introduction of therapeutic or diagnostic substances, materials or devices into a target tissue, which may be an organ, or its proximity. Specific non-limiting examples may include the insertion of brachitherapy needles or rods, or the insertion of a controlled release chemotherapy capsule.

The device according to disclosed embodiments provides for a simple and efficient way or performing a number of intrusive operations while minimizing or eliminating the risk of contaminating healthy tissue with biological material carried thereto from an infected tissue by the medical instrument carrying out the intrusive procedure.

The device of the invention may also find use in non-medical applications.

According to one aspect of the invention, the device is configured as a disposable device, and thus is made from materials that are considered to be or typically used disposable, and the device is thus configured as a single use device.

According to another aspect of the invention, a part of the device is configured to be disposable, for example the sleeve, and thus the remainder of the device can be sterilized between uses, while the sleeve is replaced with a new sleeve. The sleeve may thus be made from materials that are considered to be or typically used disposable.

According to another aspect of the invention, the device is configured as a multi-use device, and thus is made from materials that enable the device to be used many times, while being sterilisable between uses.

It should be noted that the word "comprising" as used throughout the appended claims is to be interpreted to mean "including but not limited to".

Those skilled in the art to which this invention pertains will readily appreciate that numerous changes, variations and modifications can be made without departing from the scope of the invention *mutatis mutandis.*

In summary, a multi-purpose contamination protection device includes a first generally tubular member defining a lumen therethrough having a distal opening, and a second generally tubular member capable of selectively assuming a first disposition in generally overlaying and proximal relationship with respect to at least a part of said first tubular member and a second disposition in which the second tubular member is inverted inside out with respect to the first disposition and in a generally distal relationship with respect to said part of said first tubular member is disclosed. Methods of use are also disclosed.

## Claims

1. A multi-purpose contamination protection device comprising a tube member defining a lumen therethrough having a distal opening, and further comprising a sleeve member capable of selectively assuming a first disposition in generally enveloping and proximal relationship with respect to at least a part of said tube member and a second disposition in which the sleeve member is inverted inside out with respect to the first disposition and in a generally distal relationship with respect to said part of said tube member.

2. A device according to claim 1, wherein:
said tube member comprising a tube outer surface and said lumen providing open communication between said distal opening and a proximal opening thereof, said lumen adapted for enabling a suitable elongate instrument to be inserted therethrough to perform a function at or beyond said distal opening;
said sleeve member having a first end connected to said tube outer surface at a connection station and further comprising a second open end,
wherein said sleeve member is configured for being inverted inside out such as to enable the device to be operated between an inserting configuration and a retracting configuration, wherein:
in the inserting configuration said sleeve is in said first disposition and at least a portion of the sleeve extends in a generally overlaying relationship with respect to a portion of the said tube outer surface proximal to said connection station, such that a first surface of said at least one portion of said sleeve is outwardly facing with respect to said tube member, and wherein
in the retracting configuration, said sleeve is in said second disposition and said at least one portion of the sleeve is inverted to assume a position distal of said connection station, wherein said first surface is inwardly facing with respect to said tube member.

3. A device according to any one of claims 1 to 2, wherein said sleeve member is generally coaxial with said tube member.

4. A device according to any one of claims 1 to 3, wherein said tube member is substantially rigid.

5. A device according to any one of claims 1 to 3, wherein said sleeve member is substantially flexible.

6. A device according to claim 4, wherein said distal opening is adapted for creating a passageway in a material by means of rotating and/or pushing the tube distally into the material.

7. A device according to claim 6, wherein said distal opening comprises any one of a sharp annular edge or a serrated annular edge.

8. A device according to any one of claims 1 to 7, wherein said sleeve member is connected to an outer surface of said tube member via a suitable ring.

9. A device according to any one of claims 1 to 8, wherein in the inserting configuration the full axial length of the sleeve extends in a generally overlaying relationship with respect to a portion of the said tube outer surface proximal to said connection station.

10. A device according to any one of claims 1 to 8, wherein said device comprises a lining housing concentrically mounted on said tube outer surface, and said sleeve is in the form of a deployable lining anchored at said housing.

11. A device according to claim 10, wherein said lining comprises an anchoring flange adapted for anchoring the lining at a proximal position as the device is inserted in a distal direction.

12. A device according to claim 11, wherein the lining is adapted for being progressively deployed as the device is inserted in a distal direction.

13. A device according to claim 10, wherein in said inserting configuration, at least a portion of said lining is accommodated in said lining housing in a folded configuration.

14. A device according to any one of claims 1 to 13, further comprising an arrangement for preventing proximal sliding of the sleeve with respect to a passageway into which the device is inserted.

15. A device according to claim 14, wherein said arrangement comprises suitable projections projecting in a generally proximal direction and adapted to anchor into said passageway.

16. A device according to claim 14, wherein said arrangement comprises any one of fiber or adhesives.

17. A device according to any one of claims 1 to 16, wherein said tube member may be made from any one of:
stainless steel, Titanium, plastics, carbon fiber, composite materials and synthetic materials.

18. A device according to any one of claims 1 to 17, wherein said sleeve may be made from any one of:
silicone, Latex, mesh fibers and various synthetic materials.

19. A device according to any one of claims 1 to 18, particularly adapted for use with a biopsy medical instrument.

20. A device according to any one of claims 1 to 19, particularly adapted for enabling any one of a syringe needle, a biopsy needle, and the like to be inserted through said lumen.

21. A device according to any one of claims 1 to 20, wherein said sleeve comprises a sedative or disinfection material.
